# EUROPÄISCHE PATENTANMELDUNG

(11) **EP 0 775 497 A2**
(43) Veröffentlichungstag der Anmeldung: **28.05.1997**
(21) Anmeldenummer: 96115332.7
(22) Anmeldetag: 25.09.1996
(51) Int. Cl.: A61L 33/00

(54) **Oberflächen mit antithrombischen Eigenschaften**

(30) Priorität: 25.11.1995 DE 19543936
(71) Anmelder: HÜLS AKTIENGESELLSCHAFT, 45764 Marl (DE)
(72) Erfinder: Hill, Frank, Dr., 40822 Mettmann (DE); Schindler, Fritz, Dr., 45879 Gelsenkirchen (DE)

(57) **Zusammenfassung**

Die Erfindung betrifft Gegenstände sowie Werkstoffe mit antithrombischen Oberflächeneigenschaften, die durch das Beschichten mit einer Zubereitung erhältlich sind, wobei die Zubereitung organofunktionelle Silane und Fluororganosilane und/oder deren Hydrolysate und/oder deren Kondensationsprodukte enthält. Ferner werden die Verfahren zur Herstellung von Gegenständen sowie Werkstoffen mit antithrombischen Eigenschaften sowie die Verwendung besagter Zubereitungen hierfür offenbart.

## Beschreibung

Die Erfindung betrifft Gegenstände sowie Werkstoffe, deren Oberflächen antithrombische Eigenschaften besitzen, Verfahren zu deren Herstellung und die Verwendung der Zubereitungen hierfür.

Für viele medizintechnische Produkte und Geräte - vor allem für solche, die mit Blut in direkten Kontakt gelangen - werden antithrombische Oberflächeneigenschaften gefordert, d. h. Oberflächen mit hämokompatiblen Eigenschaften bzw. Oberflächen, die die Blutgerinnung nicht auslösen.

Mehrere Möglichkeiten zur Erzeugung antithrombischer Materialoberflächen sind bereits bekannt, u. a.:
- Exponierung von Sulfonat- und anderen anionischen Gruppen an der Materialoberfläche:
   Pure & App. Chem. Vol. 56, No. 10, 1335 - 1344 (1984)
- Coating der Oberfläche mit Heparin: Trans. Am. Soc. Artif. Intern. Organs 15, 7 - 15 (1969)
- Coating der Oberfläche mit Phosphorylcholin: Polymer Journal, Vol. 24, 1259 - 1269 (1992); Chemistry in Britain, März-Heft, 253 - 256 (1992)
- Propfung der Kunststoffoberfläche mit Polyethylenoxid: Trends in Polymer Sci. 2, 20 - 25 (1994)

Diesen bekannten Verfahren ist gemeinsam, daß sie sich aus Kostengründen und/oder wegen unzureichender Wirksamkeit nicht durchsetzen konnten, so daß eine kostengünstige Methode zur Erzeugung wirksamer antithrombischer Materialoberflächen noch nicht verfügbar ist.

Die DE-OS 41 18 184 offenbart eine Beschichtungszusammensetzung auf der Basis von Polykondensaten von einer oder mehreren hydrolysierbaren Verbindungen mit mindestens einem Element M aus den Hauptgruppen III bis V und den Nebengruppen II bis IV des Periodensystems der Elemente, beispielsweise B, Al, Si, Ti, Zr, u. a. m. , wobei zumindest ein Teil dieser Verbindungen neben hydrolysierbaren Gruppen A, wie beispielsweise Halogene, Alkoxygruppen mit 1 bis 4 C-Atomen, Aryloxygruppen, Acryloxygruppen, Methacryloxygruppen, Alkylcarbonylgruppen, auch nicht hydrolysierbare kohlenstoffhaltige Gruppen B, beispielsweise Alkylgruppen mit 1 bis 4 C-Atomen, Alkenylgruppen mit 2 bis 4 C-Atomen, Alkinylgruppen, Arylgruppen sowie zuvor genannten Gruppen mit Substituenten, wie Halogene, Hydroxy-, Alkoxy-, Epoxi-, Amino-, Aminoalkylgruppen, aufweist und das Gesamt-Molverhältnis von Gruppen A zu Gruppen B in den zugrundeliegenden monomeren Ausgangsverbindungen 10 : 1 bis 1 : 2 beträgt, wobei im wesentlichen 0,1 bis 100 Mol.-% der Gruppen B Gruppen B' sind, die durchschnittlich 2 bis 30 Fluoratome aufweisen, die an ein oder mehrere aliphatische Kohlenstoffatome gebunden und die durch mindestens zwei Atome von M getrennt sind.

Die Herstellung einer Beschichtungszusammensetzung gemäß DE-OS 41 18 184 erfolgt in der Art, daß man zunächst alle oder einen Teil der Ausgangsverbindungen, ohne die der Gruppe B', durch Zugabe von Wasser hydrolysiert oder vorkondensiert, dann die Ausgangsverbindung der Gruppe B' zugibt und nach Reaktion derselben mit der bereits vorhandenen vorhydrolysierten Ausgangsverbindung bzw. Vorkondensaten durch Zugabe von weiterem Wasser und gegebenenfalls der restlichen bzw. übrigen Ausgangsverbindungen die Hydrolyse und Kondensation der anwesenden Spezies bis zum Erhalt eines für eine Beschichtung geeigneten Systems weiterführt. Die Zugabe der Ausgangsverbindungen mit Gruppen B', wie oben dargelegt, erfolgt dabei erst, wenn der Wassergehalt des Systems nicht mehr als 5 Gew.-%, bezogen auf das Gewicht des Gesamtsystems ohne gegebenenfalls eingesetzte Lösemittel, beträgt und nicht mehr als 50 % der theoretisch möglichen Gruppen M-OH im System vorliegen.

Die DE-OS 41 18 184 lehrt ferner die Verwendung der Beschichtungszusammensetzungen für solche Gegenstände, bei denen eine schmutz-, wasser- sowie ölabweisende Oberfläche erwünscht ist, insbesondere zur Beschichtung von Gläsern.

Der EP-A 0 171 493 sind Lackzusammensetzungen und ein Verfahren zur Herstellung kratzfester Beschichtungen zu entnehmen. Ferner lehrt die DE-PS 38 36 815 die Herstellung kratzfester Materialien.

Der Erfindung lag die Aufgabe zugrunde, Oberflächen von Gegenständen sowie Werkstoffen in einfacher und wirtschaftlicher Weise mit antithrombischen Eigenschaften auszustatten.

Die Aufgabe wird erfindungsgemäß entsprechend der Angaben der Patentansprüche gelöst.

Überraschenderweise wurde gefunden, daß man Gegenstände sowie Werkstoffe durch Beschichten mit einer Zubereitung, die organofunktionelle Silane und Fluororganosilane und/oder deren Hydrolysate und/oder deren Kondensationsprodukte enthält, in vergleichsweise einfacher und wirtschaftlicher Weise herstellen und so mit antithrombischen Eigenschaften ausstatten kann. Vorzugsweise besitzen die hier eingesetzten Silane mindestens eine Alkoxygruppe, besonders bevorzugt sind solche mit 1 bis 4 C-Atomen.

Gegenstand der vorliegenden Erfindung ist daher ein Verfahren zur Herstellung von Gegenständen sowie Werkstoffen mit antithrombischen Eigenschaften, wobei man die Oberfläche eines Gegenstandes oder eines Werkstoffes mit einer Zubereitung beschichtet, die organofunktionelle Silane und Fluororganosilane und/oder deren Hydrolysate und/oder deren Kondensationsprodukte enthält.

Im allgemeinen können hier solche fluororganosilanhaltigen Zubereitungen, wie sie aus der DE-OS 41 18 184 bekannt sind, für das erfindungsgemäße Verfahren verwendet werden.

In der Regel verfährt man bei der Herstellung der Zubereitungen, die bei den erfindungsgemäßen Verfahren geeigneterweise eingesetzt werden, so, daß man alle oder einen Teil der Ausgangsverbindungen, zunächst ohne die der Gruppe B', durch Zugabe von Wasser hydrolysiert oder vorkondensiert. Dann erfolgt geeigneterweise die Zugabe der Ausgangsverbindung der Gruppe B'. Nach Reaktion der zugegebenen Komponenten mit den bereits vorhandenen vorhydrolysierten Ausgangsverbindungen bzw. Vorkondensaten kann durch Zugabe von weiterem Wasser und gegebenenfalls der restlichen bzw. übrigen Ausgangsverbindungen die Hydrolyse und Kondensation der anwesenden Spezies bis zum Erhalt eines für eine Beschichtung geeigneten Systems weitergeführt werden. Hydrolyse und Vorkondensation werden üblicherweise unter Rühren und bei Raumtemperatur durchgeführt, die Umsetzung kann aber auch bei einer höheren Temperatur erfolgen. Die Zugabe der Ausgangsverbindungen mit Gruppen B', erfolgt geeigneterweise erst, wenn der Wassergehalt des Systems nicht mehr als 5 Gew.-%, bezogen auf das Gewicht des Gesamtsystems ohne gegebenenfalls eingesetzte Lösemittel, beträgt und nicht mehr als 50 % der theoretisch möglichen Gruppen M-OH im System vorliegen, wobei der pH-Wert geeigneterweise zwischen 3 und 9 liegt, vorzugsweise bei einem pH-Wert zwischen 4 und 6,5.

Beispiele für bevorzugte organofunktionelle Silane mit Gruppen B sind:
Si(OCH₃)₄, Si(OC₂H₅)₄, Si(OC₃H₇)₄, Si(OC₄H₉)₄, SiCl₄, HSiCl₃, Si(OOCCH₃)₄, CH₃SiCl₃, CH₃Si(OC₂H₅)₃, C₂H₅SiCl₃, C₂H₅Si(OC₂H₅)₃, C₃H₇Si(OCH₃)₃, C₆H₅Si(OCH₃)₃, C₆H₅Si(OC₂H₅)₃, Cl(CH₂)₃Si(OCH₃)₃, (CH₃)₂SiCl₂, (CH₃)₂Si(OCH₃)₂, (CH₃)₂Si(OC₂H₅)₂, (CH₃)₂Si(OH)₂, (C₆H₅)₂SiCl₂, (C₆H₅)₂Si(OCH₃)₂, (C₆H₅)₂Si(OC₂H₅)₂, (i-C₃H₇)₃SiOH, CH₂=CHSi(OOCCH₃)₃, CH₂=CHSiCl₃, CH₂=CHSi(OCH₃)₃, CH₂=CHSi(OC₂H₅)₃, CH₂=CHSi(OC₂H₄OCH₃)₃, CH₂=CHCH₂Si(OCH₃)₃, CH₂=CHCH₂Si(OC₂H₅)₃, CH₂=CHCH₂Si(OOCCH₃)₃, CH₂=C(CH₃)COO(CH₂)₃Si(OCH₃)₃, CH₂=C(CH₃)COO(CH₂)₃Si(OC₂H₅)₃, CH₂=CHCOO(CH₂)₃Si(OC₂H₅)₃, H₂NC₆H₄Si(OC₂H₅)₃, H₂N(CH₂)₃Si(OCH₃)₃, H₂N(CH₂)₃Si(OC₂H₅)₃, H₂N(CH₂)₃Si(CH₃)(OC₂H₅)₂, H₂N(CH₂)₂NH(CH₂)₃Si(CH₃)(OCH₃)₂, H₂N(CH₂)₂NH(CH₂)₃Si(OCH₃)₃, H₃CNH(CH₂)₃Si(OCH₃)₃, NC(CH₂)₃Si(OC₂H₅)₃, HS(CH₂)₃Si(OCH₃)₃, HS(CH₂)₃Si(OC₂H₅)₃, HS(CH₂)₃Si(CH₃)(OCH₃)₂, H₂NCONH(CH₂)₃Si(OC₂H₅)₃,

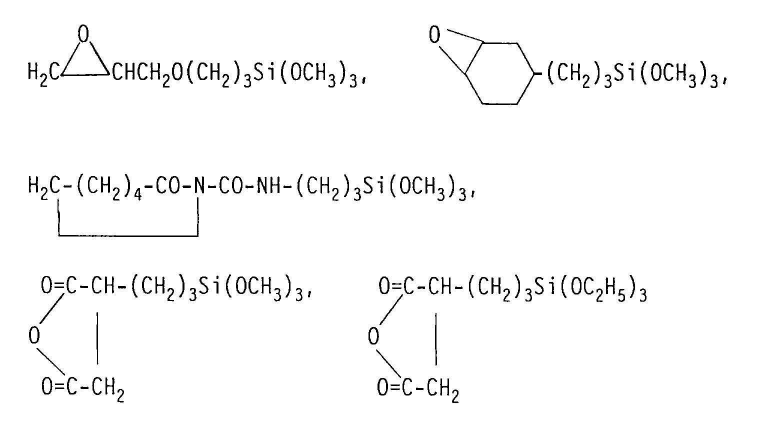

Beispiele für bevorzugte Fluororganosilane mit Gruppen B' sind:
CF₃-(CH₂)₂SiX₃, C₂F₅-(CH₂)₂SiX₃, C₄F₉-(CH₂)₂SiX₃, n-C₆F₁₃-(CH₂)₂SiX₃, n-C₈F₁₇-(CH₂)₂SiX₃, n-C₁₀F₂₁-(CH₂)₂SiX₃, wobei X hydrolysierbare Gruppen sind, insbesondere (OCH₃), (OC₂H₅) und/oder Cl.

Gegenstand der vorliegenden Erfindung ist auch die Verwendung von Zubereitungen zur Herstellung erfindungsgemäßer Gegenstände oder Werkstoffe, deren Oberflächen antithrombische Eigenschaften besitzen, wobei man eine Zubereitung einsetzt, die organofunktionelle Silane und Fluororganosilane und/oder deren Hydrolysate und/oder deren Kondensationsprodukte enthält.

Die Zubereitungen für die Herstellung erfindungsgemäßer Gegenstände oder Werkstoffe können ferner Alkoxide mit 1 bis 4 C-Atomen des Bors oder des Aluminiums oder des Zinns oder des Titans oder des Zirkons oder des Zinks oder Mischung daraus enthalten.

Vorzugsweise solche Zubereitungen, die organofunktionelle Silane und Fluororganosilane und/oder deren Hydrolysate und/oder deren Kondensationsprodukte in Verbindung mit Alkoxiden des Titans und/oder Zirkons und/oder deren Hydrolysate und/oder entsprechende Kondensationsprodukte enthalten, wobei die hier eingesetzten Silane bevorzugt mindestens eine Alkoxygruppe besitzen, werden für die Erzeugung von Oberflächen mit antithrombischen Eigenschaften verwendet.

Die besagten Zubereitungen können aber auch noch andere Bestandteile enthalten, so z. B. Lösemittel - wie Alkohole mit 1 bis 4 C-Atomen, Ether, aromatische Kohlenwasserstoffe, Ketone, Ester -; Carbonsäuren, beispielsweise Acrylsäure, Methacrylsäure; ungesättigte organische Verbindungen, wie Styrol, Nitrile; Netzhilfsmittel; Filmbildner; Füllstoffe; Färbemittel; Oxidationsinhibitoren; UV-Absorber; Katalysatoren für eine photochemisch und/oder thermisch initiierte Härtung und andere Stoffe, wie sie für Lacke bekannt sind.

Das Beschichten von Gegenständen sowie Werkstoffen mit einer Zubereitung beinhaltet bei der vorliegenden Erfindung in der Regel das Aufbringen der Zubereitung auf den Gegenstand oder Werkstoff und die Härtung der aufgebrachten Zubereitung. Unter einer aufgebrachten und ausgehärteten Zubereitung ist hier auch die fertige Beschichtung, auch Coating genannt, zu verstehen.

Das Aufbringen der Zubereitung auf einen zu beschichtenden Gegenstand oder Werkstoff erfolgt in der Regel durch Tauchen, Spritzen, Streichen oder einer anderen diesbezüglich bekannten Arbeitsweise. Üblicherweise erfolgt vor der Härtung der auf einen Gegenstand oder Werkstoff aufgebrachten Zubereitung eine Trocknungsphase, geeigneterweise bei Raumtemperatur. Die Härtung der Zubereitung kann durch eine thermisch und/oder photochemisch initiierte Polymerisation erfolgen, beispielsweise durch Bestrahlung der getrockneten Beschichtungszusammensetzung mit einer IR- oder UV-Lampe oder anderen Licht- bzw. Wärmequellen. Bei der vorliegenden Erfindung liegt die Schichtdicke eines Coatings üblicherweise im Bereich von 0,001 bis 1 mm, bevorzugt werden Schichten von 0,001 bis 0,01 mm.

Gegenstand der vorliegenden Erfindung sind ferner Gegenstände oder Werkstoffe, deren Oberflächen antithrombische Eigenschaften besitzen, die durch Beschichten mit einer Zubereitung erhältlich sind, wobei diese organofunktionelle Silane und Fluororganosilane und/oder deren Hydrolysate und/oder deren Kondensationsprodukte enthält. Vorzugsweise besitzen die hier eingesetzten Silane mindestens eine Alkoxygruppe, besonders bevorzugt sind Alkoxygruppen mit 1 bis 4 C-Atomen.

Insbesondere handelt es sich bei den erfindungsgemäßen Gegenständen um medizinische Geräte, Instrumente sowie Hilfsmittel, vorzugsweise z. B. Katheter, Blutbeutel, Dialysemembranen, künstliche Blutgefäße, Implantate, chirurgische Instrumente, wie z. B. Skalpelle, chirurgisches Nähmaterial, Stents sowie zur Blutuntersuchung eingesetzte Geräte und Gefäße.

Bei der vorliegenden Erfindung kann es sich um folgende Werkstoffe handeln: Metalle, wie z. B. Aluminium, Keramiken, wie z. B. Hydroxylapatit, Gläser, wie z. B. Borsilikatglas, Kunststoffe, wie z. B. Polyamide und/oder Naturstoffe, wie z. B. derivatisierte Cellulose. Die Aufzählung schließt ebenfalls Verbundwerkstoffe, z. B. faserverstärkte Kunststoffe, ein. Die genannten Werkstoffe können für den Einsatz im erfindungsgemäßen Verfahren in geeigneter Weise vorbehandelt werden, wie z. B. durch Ätzen (Plasmaätzen) sowie andere bekannte Verfahren zur Oberflächenbehandlung.

Gegenstand der vorliegenden Erfindung ist auch ein Verfahren zur Herstellung erfindungsgemäßer Gegenstände, indem man erfindungsgemäße Werkstoffe zur Fertigung der Gegenstände einsetzt.

Besonders überraschend bei der vorliegenden Erfindung ist auch, daß die Oberflächen der erfindungsgemäßen Gegenstände oder Werkstoffe in hervorragender Weise hämokompatible Eigenschaften - d. h. keine die Blutgerinnung auslösende Eigenschaften - besitzen, obwohl die Coatings der erfindungsgemäßen Gegenstände oder Werkstoffe vorzugsweise eine Oberflächenspannung von 10 bis kleiner 20 mN/m besitzen und damit bereits außerhalb des Bereiches von 20 bis 30 mN/m - mit einem Optimum bei 25 mN/m - liegen, der gemäß R. E. Baier der günstigste Bereich zur Vermeidung von biochemischen Vorgängen wie der Thrombusbildung ist; Journal of Colloid and Interface Science 88 (1), 296 - 297 (1982); Chemtech. 16 (3), 178 - 185 (1986).

Die vorliegende Erfindung wird durch die nachfolgenden Beispiele näher erläutert:

### Beispiele:

### Beispiel 1

Eine Aluminiumfolie (Al-Folie) mit einer Dicke von 0,3 mm wurde gemäß Beispiel 1 von DE-OS 41 18 418 gecoatet (Einsatz von 1H, 1H, 2H, 2H-Perfluoroctyltriethoxysilan als Fluororganosilan). Die Bestimmung der Oberflächenspannung der gecoateten Aluminiumfolie erfolgte nach S. Wu, J. Polymer. Sci.: Part C, No. 34, pp. 19-30 (1971), über die Messung der Randwinkel mit VE-Wasser und Dijodmethan.

| Probe | Randwinkel | | Oberflächenspannung (geometrisches Mittel) | | |
|---|---|---|---|---|---|
| | H₂O [grd] | CH₂J₂ [grd] | Gamma-D [mN/m] | Gamma-P [mN/m] | Summe [mN/m] |
| gecoatete Al-Folie aus Beispiel 1 | 101 | 78 | 17.06 | 2.03 | 19.09 |

### Beispiel 2

9 Teile Humanblut eines freiwilligen Spenders wurden mit 1 Teil Natriumcitratlösung versetzt und 5 Minuten bei 700 UpM zentrifugiert. Der trübe grau-gelbliche Überstand (= Thrombocyten-reiches Plasma) wurde mit einer Spritze aufgezogen und 1 ml davon wurde in ein Polycarbonatröhrchen zu 8 x 8 mm großen Stücken der erfindungsgemäß beschichteten Aluminiumfolie gegeben. Es wurde 30 Minuten bei einer Temperatur von 37 °C inkubiert. Dann wurde mit einer Pasteurpipette das Plasma abgesaugt und mit 1 ml Waschpuffer (10 mM Kaliumhydrogenphosphat, pH 7,4; 0,9 Gew.-% NaCl) gewaschen. Nach dem Absaugen des Puffers wurde mit 1 ml Fixierlösung (Waschpuffer + 1,5 Gew.-% Glutaraldehyd) versetzt und 10 Minuten inkubiert. Dann wurde erneut abgesaugt und in einer Reihe von Ethanol/Wassermischungen (30 %, 50 %, 70 %, 90 %) und Ethanol (100 %) entwässert. Es wurde im Vakuum über Nacht getrocknet und anschließend mit Gold besputtert. Die fertigen Präparate wurden im Rasterelektronenmikroskop betrachtet und mit einem Scanner dokumentiert.

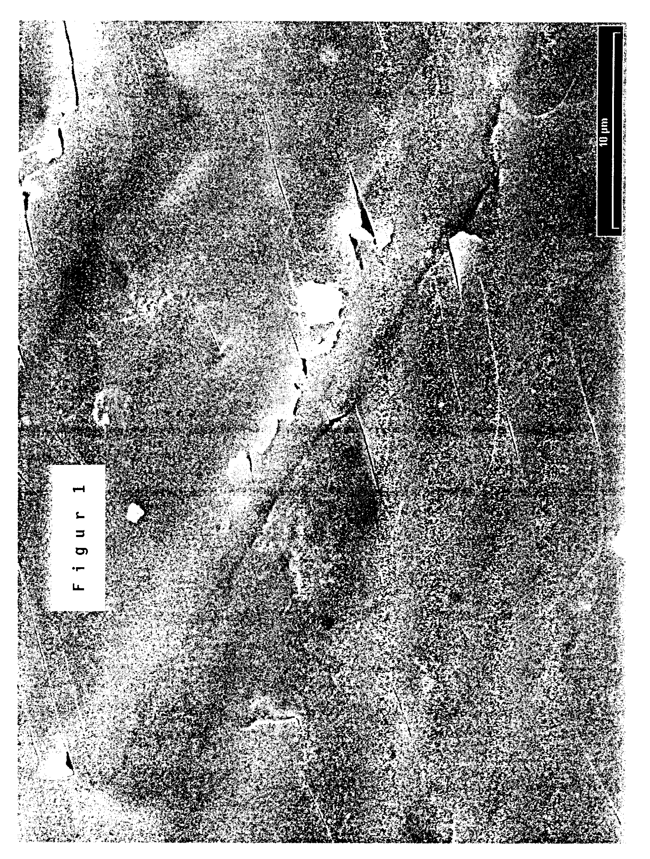

Die Figur 1 zeigt die Oberfläche einer erfindungsgemäß beschichteten Aluminiumfolie nach dem Kontakt mit Thrombocyten-reichem Humanplasma und der oben beschrieben Nachbehandlung: Es sind keine korpuskulären Ablagerungen vorhanden. Durch das erfindungsgemäße Beschichten der Aluminiumfolie ist die Oberfläche hämokompatibel geworden.

### Vergleichsbeispiel

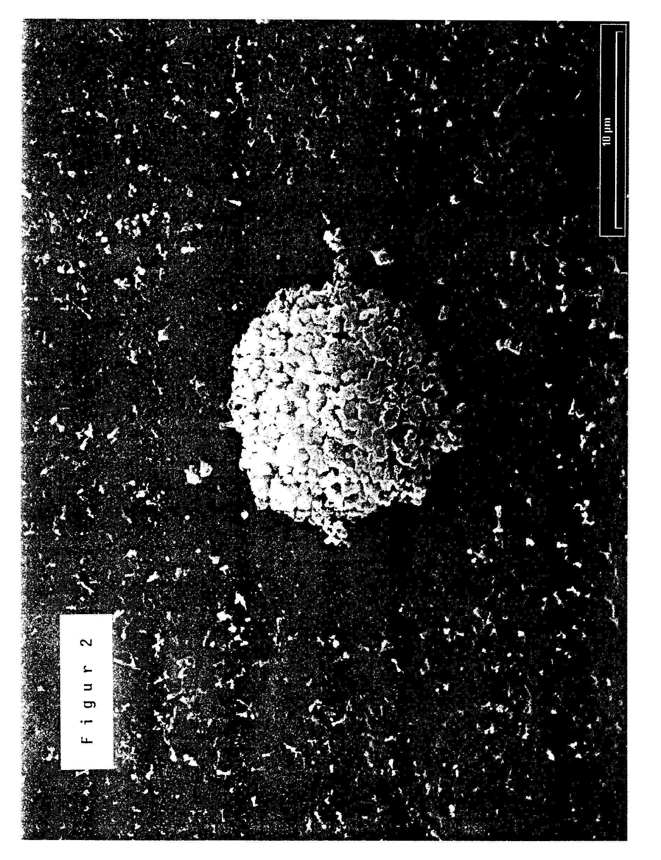

Die Figur 2 zeigt die Oberfläche einer unbeschichteten Aluminiumfolie, die ebenfalls, wie schon zuvor in Beispiel 2 beschrieben, mit Thrombocyten-reichem Humanplasma kontaktiert und nachbehandelt wurde. Figur 2 zeigt einen Mikrothrombus aus zusammengeballten Thrombocyten und anderen Blutinhaltsstoffen neben einer Vielzahl einzelner Thrombocyten. Die unbeschichtete Aluminiumfolie besitzt eine Oberfläche mit thrombogenen Eigenschaften.

### Legende zu Figur 1:

Rasterelektronenmikroskopische Aufnahme der Oberfläche einer erfindungsgemäß beschichteten Aluminiumfolie nach dem Kontakt mit Thrombocyten-reichem Humanplasma

### Legende zu Figur 2:

Rasterelektronenmikroskopische Aufnahme der Oberfläche einer unbeschichteten Aluminiumfolie nach dem Kontakt mit Thrombocyten-reichem Humanplasma

## Patentansprüche

1. Gegenstände, deren Oberflächen antithrombische Eigenschaften besitzen, erhältlich durch
Beschichten der Gegenstände mit einer Zubereitung, die organofunktionelle Silane und Fluororganosilane und/oder deren Hydrolysate und/oder deren Kondensationsprodukte enthält.

2. Gegenstände nach Anspruch 1,
dadurch gekennzeichnet,
daß die Gegenstände medizinische Geräte, Instrumente, Hilfsmittel oder Vorrichtungen sind.

3. Gegenstände nach einem der Ansprüche 1 oder 2,
dadurch gekennzeichnet,
daß die Gegenstände Katheter, Dialysemembranen, Blutbeutel, künstliche Blutgefäße, Implantate, chirurgische Instrumente, Skalpelle, chirurgisches Nähmaterial, Stents und/oder zur Blutuntersuchung eingesetzte Vorrichtungen sind.

4. Gegenstände nach mindestens einem der Ansprüche 1 bis 3,
dadurch gekennzeichnet,
daß die Fluororganosilane der Formel CF₃-(CH₂)₂SiX₃, C₂F₅-(CH₂)₂SiX₃, C₄F₉-(CH₂)₂SiX₃, n-C₆F₁₃-(CH₂)₂SiX₃, n-C₈F₁₇-(CH₂)₂SiX₃ oder n-C₁₀F₂₁-(CH₂)₂SiX₃ entsprechen und X eine hydrolysierbare Gruppe aus der Reihe -OCH₃, -OC₂H₅, -Cl darstellt.

5. Gegenstände nach mindestens einem der Ansprüche 1 bis 4,
dadurch gekennzeichnet,
daß die Zubereitung Alkoxide mit 1 bis 4 C-Atomen des Bors oder des Aluminiums oder des Zinns oder des Zinks oder des Titans oder des Zirkons oder Mischungen daraus und/oder deren Hydrolysate und/oder deren Kondensationsprodukte enthält.

6. Gegenstände nach mindestens einem der Ansprüche 1 bis 5,
dadurch gekennzeichnet,
daß deren Coating eine Oberflächenspannung von 10 bis kleiner 20 mN/m aufweist.

7. Werkstoffe, deren Oberflächen antithrombische Eigenschaften besitzen, erhältlich durch
Beschichten der Werkstoffe mit einer Zubereitung, die organofunktionelle Silane und Fluororganosilane und/oder deren Hydrolysate und/oder deren Kondensationsprodukte enthält.

8. Werkstoffe nach Anspruch 7,
dadurch gekennzeichnet,
daß die Werkstoffe Gläser, Metalle, Keramiken, Kunstoffe und/oder Naturstoffe sind.

9. Werkstoffe nach einem der Ansprüche 7 oder 8,
dadurch gekennzeichnet,
daß die Fluororganosilane der Formel CF₃-(CH₂)₂SiX₃, C₂F₅-(CH₂)₂SiX₃, C₄F₉-(CH₂)₂SiX₃, n-C₆F₁₃-(CH₂)₂SiX₃, n-C₈F₁₇-(CH₂)₂SiX₃ oder n-C₁₀F₂₁-(CH₂)₂SiX₃ entsprechen und X eine hydrolysierbare Gruppe aus der Reihe -OCH₃, -OC₂H₅, -Cl darstellt.

10. Werkstoffe nach mindestens einem der Ansprüche 7 bis 9,
dadurch gekennzeichnet,
daß die Zubereitung Alkoxide mit 1 bis 4 C-Atomen des Bors oder des Aluminiums oder des Zinns oder des Zinks oder des Titans oder des Zirkons oder Mischungen daraus und/oder deren Hydrolysate und/oder deren Kondensationsprodukte enthält.

11. Werkstoffe nach mindestens einem der Ansprüche 7 bis 10,
dadurch gekennzeichnet,
daß deren Coating eine Oberflächenspannung von 10 bis kleiner 20 mN/m aufweist.

12. Verfahren zur Herstellung von Gegenständen, deren Oberflächen antithrombische Eigenschaften besitzen, nach den Ansprüchen 1 bis 6,
dadurch gekennzeichnet,
daß man die Oberfläche eines Gegenstandes mit einer Zubereitung beschichtet, die organofunktionelle Silane und Fluororganosilane und/oder deren Hydrolysate und/oder deren Kondensationsprodukte enthält.

13. Verfahren nach Anspruch 12,
dadurch gekennzeichnet,
daß man die Oberfläche eines Gegenstandes mit einer Zubereitung beschichtet, die Alkoxide des Titans und/oder Zirkons und organofunktionelle Silane und Fluororganosilane und/oder deren Hydrolysate und/oder deren Kondensationsprodukte enthält.

14. Verfahren zur Herstellung von Werkstoffen, deren Oberflächen antithrombische Eigenschaften besitzen, nach den Ansprüchen 7 bis 11, dadurch gekennzeichnet,
daß man die Oberfläche eines Werkstoffes mit einer Zubereitung beschichtet, die organofunktionelle Silane und Fluororganosilane und/oder deren Hydrolysate und/oder deren Kondensationsprodukte enthält.

15. Verfahren nach Anspruch 14,
dadurch gekennzeichnet,
daß man die Oberfläche eines Werkstoffes mit einer Zubereitung beschichtet, die Alkoxide des Titans und/oder Zirkons und organofunktionelle Silane und Fluororganosilane und/oder deren Hydrolysate und/oder deren Kondensationsprodukte enthält.

16. Verfahren zur Herstellung von Gegenständen nach den Ansprüchen 1 bis 6,
dadurch gekennzeichnet,
daß man die Werkstoffe nach den Ansprüchen 7 bis 11 zur Fertigung der Gegenstände einsetzt.

17. Verwendung von Zubereitungen zur Herstellung von Gegenständen, deren Oberflächen antithrombische Eigenschaften besitzen, nach den Ansprüchen 1 bis 6,
dadurch gekennzeichnet,
daß man eine Zubereitung einsetzt, die organofunktionelle Silane und Fluororganosilane und/oder deren Hydrolysate und/oder deren Kondensationsprodukte enthält.

18. Verwendung von Zubereitungen zur Herstellung von Werkstoffen, deren Oberflächen antithrombische Eigenschaften besitzen, nach den Ansprüchen 7 bis 11,
dadurch gekennzeichnet,
daß man eine Zubereitung einsetzt, die organofunktionelle Silane und Fluororganosilane und/oder deren Hydrolysate und/oder deren Kondensationsprodukte enthält.
